# EUROPEAN PATENT APPLICATION

(11) **EP 4 413 870 A1**
(43) Date of publication of application: **14.08.2024**
(21) Application number: 23155686.1
(22) Date of filing: 08.02.2023
(51) Int. Cl.: A23L 7/104, C12P 7/26

(54) **A PROCESSS FOR PREPARING A FERMENTED PLANT-BASED PRODUCT COMPRISING DIACETYL**

(71) Applicant: Arla Foods Amba, 8260 Viby J (DK)
(72) Inventor: Madsen,, John Charles, Viby J, Denmark (DK); Pallesen,, Lotte Hansen, Viby J, Denmark (DK); Lucena,, Daniella, Viby J, Denmark (DK)
(74) Representative: Potter Clarkson

(57) **Abstract**

The present invention relates to a process for preparing a fermented plant-based product comprising diacetyl, the products obtained by the process and the use thereof. In particular the invention relates to a process for preparing a fermented plant-based product using a plant base, at least one lactic acid bacteria and a substrate for obtaining increased diacetyl production.

## Description

### Technical field of the invention

The present invention relates to a process for preparing a fermented plant-based product comprising diacetyl, the products obtained by the process, and the use thereof. In particular, the invention relates to a process for preparing a fermented plant-based product using a plant base, a mixture of lactic acid bacteria, and a substrate for obtaining increased diacetyl production.

### Background of the invention

The demand for plant-based food products, as an alternative to dairy products, is increasing. In particular, the consumers demand plant-based alternatives with sensory properties similar to their non plant-based counterparts. Sensory properties include smell, texture, flavour, and appearance. Taste refers to the senses inside the mouth including the tongue. Aroma occurs inside the nose and relates specifically to the sense of smell, whereas flavour is when taste and aroma converge.

Although the number of available plant-based products continues to increase, the formulation of plant-based products remains a challenge. Plant-based alternatives that resemble traditional dairy-based products like milk, yoghurt, cheese, and frozen desserts struggle to obtain similar sensory characteristics as their dairy-based equivalents. In particular plant-based spreadable with a sufficient butter-like flavour are lacking from the selection of plant-based spreadable products currently available. One of the molecules responsible for the butter-like flavour is diacetyl, also known as butanedione or butane-2,3-dione.

Diacetyl may be formed as a by-product during lactic acid bacteria fermentation. Lactic acid bacteria have been used in food fermentation for centuries and fermentation constitutes one of the earliest known food preserving techniques. In particular lactic acid bacteria are widely used in the food industry in fermentation of food products such as dairy products, meat, fish, vegetables, fruits, and cereal products. In addition to extending the shelf-life of food products, lactic acid fermentation is often applied to affect the sensory properties of food products. Although diacetyl may be formed during fermentation, it still remains a challenge to control the production of diacetyl (e.g. such as the amount of diacetyl) during fermentation.

Based on the above, it would be advantageous to provide a process for obtaining a fermented plant-based product with a butter-like flavour similar to that of dairy products to meet the increasing demand for plant-based food alternatives with sensory characteristics similar to those of dairy products.

### Summary of the invention

The present invention relates to a process for preparing a fermented plant-based product comprising diacetyl, the products obtained by the process, and the use thereof. In particular the invention relates to a process for preparing a fermented plant-based product using a plant base, a mixture of lactic acid bacteria, and a substrate for obtaining increased diacetyl production in the fermented plant-based product. The fermented plant-based product is suitable for use as a flavour enhancer in plant-based spreadable products.

Thus, an object of the present invention relates to the provision of a process for preparing a fermented plant-based product comprising diacetyl and the products obtained by the process. In particular it is an object of the present invention to provide a process to obtain a fermented flavour-enhancing product to be used in e.g. a plant-based spreadable product so as to mimic the flavour of butter and other spreadable dairy-based products.

Thus, an aspect of the present invention relates to a process for preparing a fermented plant-based product comprising diacetyl, wherein the process comprises the steps of:
a) provision of a mixture comprising:
   i. at least one plant base, and
   ii. at least one lactic acid bacterium,
b) a first fermentation of the mixture until the pH of the mixture is in the range of 4.5 -6.5,
c) addition of a substrate to the mixture of step b), and
d) a second fermentation of the mixture of step c) to provide a fermented plant-based product comprising diacetyl.

Another aspect of the present to invention relates to a fermented plant-based product comprising diacetyl obtainable by a process as described herein.

A further aspect of the present invention relates to a fermented plant-based product comprising at least 0.1 ppm diacetyl.

A further aspect of the present invention relates to a food product comprising the fermented plant-based product as described herein.

Yet another aspect of the present to invention relates to a process for producing a plant-based spreadable product comprising diacetyl, wherein the process comprises the steps of:
a) providing an emulsion comprising:
   i. a fat phase,
   ii. a water phase comprising the fermented plant-based product as described herein,
b) heating the fat phase and water phase separately to at least 40°C,
c) mixing the fat phase and water phase to form the emulsion,
d) heating the emulsion,
e) cooling the emulsion to at least 2 to 20°C, preferably 10°C to obtain the plant-based spreadable product comprising diacetyl, obtaining a plant-based spreadable product.

A further aspect of the present invention relates to a plant-based spreadable product obtainable by a process as described herein.

Still, a further aspect of the present invention relates to a plant-based spreadable product comprising diacetyl and/or more ingredients selected from the group consisting of oils, fats, water, a plant-base, salt, an acid, one or more colouring agents, and any combination thereof.

Another aspect of the present invention relates to a use of the fermented plant-based product as described herein in the preparation of a food product as described herein.

The present invention will in the following be described in more detail.

### Detailed description of the invention

The inventors of the present invention have developed a process for preparing a fermented plant-based product comprising diacetyl, which provides enhanced butter-like flavour to plant-based food products similar to that of dairy-based products.

### Definitions

Prior to outlining the present invention in more details, a set of terms and conventions is first defined:

### Diacetyl

In the present context, the term "diacetyl" is to be understood as an organic compound having the chemical formula (CH₃CO)₂. Diacetyl is one of the main aroma compounds that provides butter flavour. In the present context the term "diacetyl" is herein used interchangeably with the terms butanedione or butane-2,3-dione. Diacetyl can be measured by headspace Gas Chromatography/Mass Spectrometry (Headspace GC/MS).

### Substrate

In the present context, the terms "substrate" is to be understood as a compound provided to at least one lactic acid bacteria to boost diacetyl production during fermentation. The substrate may be pyruvate, pyruvate acid or salts thereof and/or citrate, citric acid or salts thereof, in particular e.g. sodium citrate.

### Butter-like flavour

In the present context, the term "butter-like flavour" is to be understood as the aromatic flavour derived from diacetyl.

### Fermentation mixture

In the present context, the term "fermentation mixture" is to be understood as a mixture comprising at least one plant base and at least one lactic acid bacterium. A fermentation mixture in the present invention can be either with or without a substrate (i.e. pyruvate, pyruvate acid or salts thereof and/or citrate, citric acid or salts thereof).

### First fermentation

In the present context the term "first fermentation" is to be understood as the part of the fermentation process performed before the addition of a substrate.

### Second fermentation

In the present context the term "second fermentation" is to be understood as the part of the fermentation process performed after the addition of a substrate.

### Lactic acid bacteria

As used herein, the term "lactic acid bacterium" or "lactic acid bacteria" designates a gram-positive, microaerophilic or anaerobic bacterium, which ferments sugars with the production of acids including lactic acid as the predominantly produced acid, acetic acid and propionic acid.

For the purpose of the present invention the most relevant lactic acid bacteria originate from the genera *Lactobacillus, Lacticaseibacillus, Streptococcus, Bifidobacterium, Lactococcus, Leuconostoc* and combinations thereof. Some of these may produce diacetyl as a by-product during fermentation. The lactic acid bacteria may be supplied as either frozen or freeze-dried cultures for bulk starter propagation or as so-called "Direct Vat Set" (DVS) cultures, intended for direct inoculation into the fermenter. Such may be referred to as "starter cultures" or "starters".

The inventors of the present invention have surprisingly succeeded in developing a process for preparing a fermented plant-based product comprising diacetyl. The fermented plant-based product comprising diacetyl may be applied to introduce a butter-like flavour in plant-based food products, such as spreadable products.

The inventors of the present invention have successfully demonstrated that a determining factor for boosting the amount of diacetyl produced during fermentation with at least one lactic acid bacterium is the substrate and the timing of adding the substrate during fermentation. The timing may for example be a certain point in time after initiating fermentation and/or when a certain pH is reached during fermentation.

Thus, an aspect of the present invention relates to a process for preparing a fermented plant-based product comprising diacetyl, wherein the process comprises the steps of:
a) provision of a mixture comprising:
   i. at least one plant base, and
   ii. at least one lactic acid bacteria,
b) a first fermentation of the mixture until the pH of the mixture is in the range of 4.5 - 6.5,
c) addition of a substrate to the mixture of step b), and
d) a second fermentation of the mixture of step c) to provide a fermented plant-based product comprising diacetyl.

The inventors have found that the addition of a substrate to the fermentation mixture during fermentation is key in achieving an increased diacetyl yield. In the present context the terms "increasing diacetyl yield, increasing the amount of diacetyl and/or increasing diacetyl production" are to be understood as an increase in the amount of diacetyl obtained (i) by the process for preparing a fermented plant-based product comprising diacetyl according to the present invention when compared to the amount of diacetyl obtained (ii) in an identical process wherein no substrate is added when the pH, during fermentation, reaches 4.5 - 6.5. The increase in the amount of diacetyl obtained by the present invention should be preferably be at least 1 w/w%, such as at least 5 w/w%, e.g. at least 10 w/w%, such as at least 20 w/w%, e.g. at least 30 w/w%, such as at least 40 w/w%, e.g. at least 50 w/w%, such as at least 60 w/w%, e.g. at least 70 w/w%, such as at least 60 w/w%, e.g. at least 70 w/w%, such as at least 80 w/w%, e.g. at least 90 w/w%, such as at least 100 w/w%, e.g. at least 150 w/w%, such as 200 w/w%, e.g. at least 250 w/w%, such as at least 300 w/w% when compared to the amount of diacetyl obtained by an identical process but wherein no substrate is added when the pH, during fermentation, reaches 4.5 - 6.5.

In an embodiment of the present invention the substrate is pyruvate, pyruvic acid or salts thereof and/or citrate, citric acid or salts thereof. Depending on the substrate the aromatic butter flavour from diacetyl might change. If the substrate is pyruvate, pyruvate acid or salts thereof the butter flavour from diacetyl may be perceived differently than if citrate, citric acid or salts thereof is applied as the substrate. In particular, the use of citrate as substrate in the fermentation process of the present inventions is suitable in or for some applications whereas the use of pyruvate as substrate in the fermentation process of the present inventions is suitable in or for other applications. In an embodiment the substrate is preferably citrate, citric acid or salts thereof, In another embodiment the substrate is pyruvate, pyruvate acid or salts thereof. If the fermented plant-based product comprising diacetyl is to be applied in a plant-based spreadable product it may be preferred that the substrate is citrate, citric acid or salts thereof.

In an embodiment of the present invention the substrate is added in a concentration ranging from 0.01 to 0.50 g/L, such as 0.05 to 0.45 g/L, e.g. 0.1 to 0.40 g/L, such as 0.15 to 0.40 g/L, e.g. 0.20 to 0.35 g/L, such as 0.25 to 0.30 g/L and preferably 0.05 to 0.15 g/L.

For at least one lactic acid bacterium to grow and produce metabolites (such as lactic acid and/or diacetyl), sugar is needed in the fermentation mixture. It may therefore be contemplated that the mixture in step a) has a total sugar content in the range of 0.1 to 20 w/w%, such as 0.5 to 9.5 w/w%, e.g. 1 to 9 w/w%, such as 1.5 to 8.5 w/w%, e.g. 2 to 8 w/w%, such as 2.5 to 7.5 w/w%, e.g. 3 to 7 w/w%, such as 3.5 to 7.5 w/w%, e.g. 4 to 7 w/w%, such as 4.5 to 6.5 w/w%, e.g. 5 to 6 w/w%, such as 4.5 to 9.5 w/w% and preferably 2 w/w% to 3.2 w/w% total sugar. It may be preferred to have a high total sugar content in the mixture of step a) to ensure that residual sugar is present in the fermented plant-based product comprising diacetyl obtained by the present invention.

In the present context, the term "sugar content" is to be understood as the percentage of sugar present in the mixture in step a) of the process of the invention and/or in the fermented plant-based product comprising diacetyl. The sugar content may originate fully or partly from the plant base used in the fermentation process or which is part of the fermented plant-based product comprising diacetyl. The total sugar content may be measured by simple Brix analysis and specific sugars can be determined by chromatographical methods, e.g. ion chromatography. In the present context sugar is to be understood as saccharides, but may also include starch and cellulose, as some lactic acid bacteria may be able to degrade starch. Sugars preferably include monosaccharides, disaccharides, oligosaccharides, and polysaccharides.

In the present context, the terms "plant base" and "plant-based" are to be understood as the starting raw material that is subjected to the fermentation process of the present invention or which is part of the product(s) of the present invention. This raw material may comprise plant parts derived from cereals, seeds, nuts, vegetables, stone fruits, pulses and combinations thereof. The selection of plant base impacts the taste of the end product - i.e. the fermented plant-based product comprising diacetyl and the selection of the plant base may therefore be selected depending on e.g. the application of the fermented plant-based product comprising diacetyl.

Therefore, in one embodiment the present invention relates to the process or product as described herein, wherein the plant base is selected from the group consisting of cereals, seeds, nuts, vegetables, stone fruits, pulses and combinations thereof. An further embodiment of the present invention relates to the process or product as described herein, wherein the plant base is selected from the group consisting of oat, wheat, rye, barley, corn, rice, quinoa, chia, rapeseed, almond, coconut, carrot, pumpkin and combinations thereof.

Yet, a further embodiment of the present invention relates to the process or product as described herein, wherein the plant base is cereal. A further embodiment of the present invention relates to the process as described herein, wherein the cereal plant base is selected from the group consisting of oat, wheat, rye, barley, corn, rice, quinoa and combinations thereof.

A specific embodiment of the present invention relates to the process or product as described herein, wherein the cereal is oat. Although the flavour of the selected plant base changes through fermentation it still impacts the flavour of the end product (e.g. the fermented plant-based product comprising diacetyl). Thus, oat may in particular be preferred as a plant base in the process of the present invention or in the product(s) of the present invention if the fermented plant-based product comprising diacetyl is to be applied in a plant-based spreadable.

It may be preferred that the mixture of step a) comprises a total sugar content in the range of 0.1 to 20 w/w%, such as 0.5 to 9.5 w/w%, e.g. 1 to 9 w/w%, such as 1.5 to 8.5 w/w%, e.g. 2 to 8 w/w%, such as 2.5 to 7.5 w/w%, e.g. 3 to 7 w/w%, such as 3.5 to 7.5 w/w%, e.g. 4 to 7 w/w%, such as 4.5 to 6.5 w/w%, e.g. 5 to 6 w/w%, such as 4.5 to 9.5 w/w% and preferably 2 w/w% to 3.2 w/w% total sugar.

It may be preferred that the plant-base of step a) comprises a total sugar content in the range of 0.1 to 20 w/w%, such as 0.5 to 9.5 w/w%, e.g. 1 to 9 w/w%, such as 1.5 to 8.5 w/w%, e.g. 2 to 8 w/w%, such as 2.5 to 7.5 w/w%, e.g. 3 to 7 w/w%, such as 3.5 to 7.5 w/w%, e.g. 4 to 7 w/w%, such as 4.5 to 6.5 w/w%, e.g. 5 to 6 w/w%, such as 4.5 to 9.5 w/w% and preferably 2 w/w% to 3.2 w/w% total sugar.

The amount of plant base to be applied may depend on the at least one lactic acid bacterium or the combination of lactic acid bacteria applied and the amount of sugar in the plant base.

In another specific embodiment of the present invention the plant base is an oat plant base and the mixture of step a) comprises 5 to 20 w/w% of the oat plant base, such as 6 to 19 w/w%, e.g. 7 to 18 w/w%, such as 8 to 17 w/w%, e.g. 9 to 16 w/w%, such as 10 to 15 w/w%, e.g. 11 to 14 w/w%, such as 12 to 13 w/w% and preferably 10 to 14% w/w%.

An embodiment of the present invention relates to the process as described herein, wherein the form of the plant base is selected from the group consisting of a syrup, a concentrate, a paste, freeze dried, granular and combinations thereof. The granular plant base may be selected from the group consisting of a flour, a concentrate, an isolate and combinations thereof.

In particular the plant base may be an oat concentrate, oat syrup, an oat isolate, oat flour and combinations thereof.

Another embodiment of the present invention relates to the process as described herein, wherein the mixture comprises a liquid medium and/or wherein the liquid medium is water. The mixture in step a) may be a fermentation medium providing at least carbon, nitrogen, minerals, amino acids and vitamins to the lactic acid bacteria.

The plant base may be heated prior to adding it to the mixture in step a) of the process of the invention. Heating can aid in eliminating contaminants, such as pathogens, spoilage microorganisms and other non-desired agents in the mixture. Thus, one embodiment of the present invention relates to the process as described herein, wherein the plant base is heated to at least 75 to 145°C, such as 80 to 140°C , e.g. 85 to 135°C, such as 90 to 130°C , e.g. 95 to 125°C, such as 100 to 120°C , e.g. 105 to 115°C, such as 110 to 130°C and preferably to 95 °C prior to step a).

In a further embodiment the plant base is heated for at least 0.3 seconds and for maximum 1 hour prior to step a), such as for at least 1 second and for maximum 55 minutes, e.g. for at least 10 seconds and for maximum 50 minutes, such as for at least 20 seconds and for maximum 45 minutes, e.g. for at least 30 seconds and for maximum 40 minutes, such as for at least 40 seconds and for maximum 35 minutes, e.g. for at least 50 seconds and for maximum 30 minutes, such as for at least 1 minute and for maximum 25 minutes, e.g. for at least 5 minutes and for maximum 20 minutes, such as for at least 10 minutes and for maximum 15 minutes.

In a specific embodiment the plant base is heated to at least 75 to 145°C, preferably to 95 °C prior to step a). In another specific embodiment the plant base is heated for at least 0.3 seconds and for maximum 1 hour prior to step a).

Changing a plant base into other organoleptically acceptable products (such as a fermented plant-based product) by fermentation requires particular attributes of e.g. lactic acid bacteria. These include rapid acid production from lactose and development of suitable quantities of the volatile compounds diacetyl and acetaldehyde. Moreover, it is important that these compounds are not accompanied by off-flavours.

Selecting the optimal combination of lactic acid bacteria to be used in fermentation of dairy products and plant-based food products has therefore shown to be challenging.

To obtain increased diacetyl production during fermentation and to ensure that the desired diacetyl flavour is obtained, the inventors have found that the process for preparing a fermented plant-based product comprising diacetyl may benefit from (i) fermenting a plant base using a combination of lactic acid bacteria and (ii) adding a substrate to the fermentation process when the pH reaches 4.5 - 6.5.

The inventors surprisingly found that when adding a substrate to the fermentation process when the pH reached 4.5 - 6.5 it is possible to increase the amount diacetyl produced by one or more lactic acid bacteria when compared to an identical fermentation process but without adding a substrate when the pH reached 4.5 - 6.5. Thus, in embodiment of the present invention therefore relates to the process and product as described herein, wherein one or more of the lactic bacteria produces diacetyl.

Lactic acid bacteria species producing diacetyl add flavour complexity to the end product in a way that concentrated diacetyl likely would not. Diacetyl is not the only aroma compound produced by the lactic acid bacteria, numerous other aroma compounds are produced during fermentation, depending on the lactic acid bacteria or the combination of lactic acid bacteria applied. Therefore, combining different lactic acid bacteria genera, species and/or strains may be important to obtain the intended flavour complexity, smell, texture, and appearance of the end product (e.g. the fermented plant-based product comprising diacetyl and any products comprising the fermented plant-based product comprising diacetyl).

It may therefore be advantageous to select a mixture of lactic acid bacteria that in synergy ensures diacetyl production and the desired butter-like flavour of the fermented plant-based product comprising diacetyl. Moreover, the selection of lactic acid bacteria may depend on the desired sensory properties of the fermented plant-based product comprising diacetyl and/or of any products comprising the fermented plant-based product comprising diacetyl.

Thus, in one embodiment the present invention relates to the process and/or product as described herein, wherein the lactic acid bacteria originate from one or more genera selected from the group consisting of *Lactobacillus, Lacticaseibacillus, Streptococcus, Bifidobacterium, Lactococcus, Leuconostoc* and combinations thereof.

A further embodiment of the present invention relates to the process and product as described herein, wherein the lactic acid bacteria is selected from the group consisting of *Streptococcus thermophilus, Lactobacillus delbrueckii subsp. bulgaricus, Lacticaseibacillus paracasei, Lactobacillus acidophilus, Bifidobacterium lactis, Lactococcus lactis, Leuconostoc mesenteroides* and combinations thereof.

An embodiment of the present invention relates to the process and product as described herein, wherein the lactic acid bacteria is *Streptococcus thermophilus.*

A further embodiment of the present invention relates to the process and product as described herein, wherein the lactic acid bacteria is *Lactobacillus delbrueckii subsp. bulgaricus.*

Yet a further embodiment of the present invention relates to the process and product as described herein, wherein the lactic acid bacteria *Lacticaseibacillus paracasei.*

A further embodiment of the present invention relates to the process and product as described herein, wherein the lactic acid bacteria is *Lactobacillus acidophilus.*

Yet further embodiment of the present invention relates to the process and product as described herein, wherein the lactic acid bacteria is *Bifidobacterium lactis.*

A further embodiment of the present invention relates to the process and product as described herein, wherein the lactic acid bacteria is *Lactococcus lactis, Leuconostoc mesenteroides.*

Yet a further embodiment of the present invention relates to the process and product as described herein, wherein the lactic acid bacteria is *Leuconostoc mesenteroides.*

The selection of lactic acid bacterial also preferably depend on their growth optimum temperature. Lactic acid bacterial strains such as *Streptococcus thermophilus* and/or *Lactobacillus delbrueckii subsp. bulgaricus* are traditionally applied in yoghurt production and may be applied in the process and product(s) described herein. Thus, in an embodiment the present invention relates to the process and product(s) as described herein, wherein the lactic acid bacteria is *Streptococcus thermophilus* and/or *Lactobacillus delbrueckii subsp. bulgaricus.* Still, a further embodiment of the present invention relates to the process and product as described herein, wherein the lactic acid bacteria is *Lacticaseibacillus paracasei, Lactobacillus acidophilus* and/or *Bifidobacterium lactis.*

With respect to the present invention in may be preferred to select lactic acid bacteria capable of citrate metabolization. Likewise it may be preferred to select lactic acid bacteria capable of fast acidification and/or which are homo- or heterofermentative.

Another embodiment of the present invention relates to the process and product as described herein, wherein the mixture in step a) and/or the fermented plant-based product comprising diacetyl comprises at least two lactic acid bacterial species, preferably at least three lactic acid bacteria species, more preferably at least four lactic acid bacteria species, most preferably at least five lactic acid bacteria species. A further embodiment of the present invention relates to the process and product as described herein, wherein the mixture in step a) and/or the product(s) of the present invention comprises more than five lactic acid bacteria species. Due to phage pressure in the dairies it may be preferred to apply at least two different lactic acid bacterial species.

In a preferred embodiment of the present invention the mixture in step a) and/or the product(s) of the present invention, comprises at least *Streptococcus thermophilus, Lactobacillus delbrueckii subsp. bulgaricus, Lacticaseibacillus paracasei, Lactobacillus acidophilus and Bifidobacterium lactis.*

Applying a combination of *Streptococcus thermophilus, Lactobacillus delbrueckii subsp. bulgaricus, Lacticaseibacillus paracasei, Lactobacillus acidophilus* and *Bifidobacterium lactis* in the process of the present invention it is possible to obtain a fermented plant-based product comprising diacetyl that is e.g. suitable as a butter-like flavour enhancer in food products.

Further, it was found that the addition of a substrate selected from the group consisting of pyruvate, pyruvate acid or salts thereof and/or citrate, citric acid or salts thereof, at a pH-value in the range of 4.5 to 6.5 during the fermentation process leads to increased production of diacetyl. The combination of (i) the mixture of lactic acid bacteria, (ii) the time point during fermentation, i.e. the pH-value range for adding the substrate and (iii) the substrate itself provides a unique process capable of producing a fermented plant-based product comprising diacetyl and having a certain sensory profile. Moreover, this combination (i)-(iii) also led to the generation of a butter-like flavour of the fermented plant-based product comprising diacetyl making it suitable for inclusion in various food products such as plant-based spreadable products.

Thus, in a preferred embodiment the process for preparing a fermented plant-based product comprising diacetyl according to the present invention, comprises the steps of:
a) provision of a mixture comprising:
   i. at least one plant base, and
   ii. at least one lactic acid bacterium,
b) a first fermentation of the mixture until the pH of the mixture is in the range of 4.5 - 6.5,
c) addition of a substrate to the mixture of step b),
d) a second fermentation of the mixture of step c) to provide a fermented plant-based product comprising diacetyl,
wherein the at least one lactic acid bacterium is a combination of *Streptococcus thermophilus, Lactobacillus delbrueckii subsp. bulgaricus, Lacticaseibacillus paracasei, Lactobacillus acidophilus* and *Bifidobacterium lactis* and wherein the substrate is citrate, citric acid or salts thereof.

In another preferred embodiment the process for preparing a fermented plant-based product comprising diacetyl according to the present invention, comprises the steps of:
a) provision of a mixture comprising:
   i. at least one plant base, and
   ii. at least one lactic acid bacterium,
b) a first fermentation of the mixture until the pH of the mixture is in the range of 4.5 -6.5,
c) addition of a substrate to the mixture of step b),
d) a second fermentation of the mixture of step c) to provide a fermented plant-based product comprising diacetyl,
wherein the at least one lactic acid bacterium is a combination of *Streptococcus thermophilus, Lactobacillus delbrueckii subsp. bulgaricus, Lacticaseibacillus paracasei, Lactobacillus acidophilus* and *Bifidobacterium lactis,* wherein the substrate is citrate, citric acid or salts thereof and wherein the plant-base is oat.

Accordingly, the inventors of the present invention succeeded in developing an improved process for preparing a fermented plant-based product comprising diacetyl, suitable for use as a flavour enhancer in plant-based spreadable products.

The first fermentation is terminated when the fermentation mixture reaches a pH-value of 4.5 to 6.5. Following the addition of the substrate to the fermentation mixture the second fermentation is initiated. The second fermentation may be terminated when the pH value of the fermentation mixture (i.e. of the fermented plant-based product comprising diacetyl is below 4.

An embodiment of the present invention the first and/or second fermentation is performed at a temperature in the range of 15 to 42°C, such as 17 to 40°C, e.g. 19 to 38°C, such as 21 to 36°C, e.g. 23 to 34°C, such as 25 to 32°C, e.g. 27 to 30°C, preferably 28°C.

In another embodiment the first fermentation is performed for 1 to 9 hours, such as at least 1 hour, such as at least 2 hours, such as at least 3 hours, such as at least 4 hours, such as at least 5 hours, preferably 3-7 hours, such as 4-6 hours, and preferably about 5 hours.

In yet, another embodiment the second fermentation is performed for 2 to 25 hours, such as 5-20 hours, such as 10-20, such as 15-20, such as 18-20. For example, the second fermentation is performed for at least 2 hours, such as at least 3 hours, such as at least 4 hours, such as at least 5 hours, such as at least 6 hours, such as at least 7 hours, such as at least 8 hours, such as at least 9 hours, such as at least 10 hours, such as at least 11 hours, such as at least 12 hours, such as at least 13 hours, such as at least 14 hours, such as at least 15 hours, such as at least 16 hours. The total fermentation time should not exceed 35 hours, and it is preferred that total fermentation is performed for no longer than 24 hours.

As described above it may be preferred that the second fermentation is terminated when the pH decreases below 4.5. In preferred embodiments, the second fermentation is terminated when the pH decreases below 4.0, such as 3.8, such as 3.7, e.g. 3.6, such as 3.5, e.g. 3.4, most preferred below 3.7.

It may be advantageous to apply agitation to the fermentation process to ensure a stable supply of oxygen or air and a uniform distribution of nutrients to the lactic acid bacteria. Providing a stable supply of oxygen or air and a uniform distribution of nutrients to the lactic acid bacteria may lead to increased diacetyl production during fermentation and/or optimal conditions for production of other important metabolites (e.g. acetoin and other aroma compounds).

In the present context, the term "agitation" is to be understood as a constant movement of the fermentation mixture for a period of time to provide stable supply of oxygen or air and a uniform distribution of nutrients to the fermentation process.

An embodiment of the present invention relates to the process as described herein, wherein the first and/or the second fermentation is performed at least in part with agitation.

Another embodiment of the present invention relates to the process and/or product as described herein, wherein the fermented plant-based product comprises at least 1 ppm diacetyl. In yet an embodiment the fermented plant-based product comprises in the range of 1 to 100 ppm such as 1-95 ppm, e.g. 10-90 ppm, such as 20-80 ppm, e.g. 30-70 ppm, such as 40-60 ppm, e.g. 35-55 ppm, such as 30-50 ppm, e.g. 25-45 ppm, preferably 20-40 ppm diacetyl.

It may be advantageous to apply the fermented plant-based product comprising diacetyl to various dairy alternatives to provide a butter-like flavour similar to that of dairy products. The fermented plant-based product comprising diacetyl may be obtained by the process as described herein.

Therefore, another aspect of the present to invention relates to a fermented plant-based product comprising diacetyl obtainable by a process as described herein.

Another aspect of the present to invention relates to a fermented plant-based product comprising diacetyl.

It must be emphasised that all embodiments relating to the process for preparing a fermented plant-based product comprising diacetyl are equally relevant to the fermented plant-based product comprising diacetyl as such and/or to any food product(s) comprising the fermented plant-based product comprising diacetyl. Likewise all embodiments disclosed in respect of the process for preparing a fermented plant-based product comprising diacetyl are also relevant to the process for producing a plant-based spreadable according as disclosed herein.

A further aspect of the present invention relates to a fermented plant based product comprising at least 0.1 ppm diacetyl. In yet an embodiment the fermented plant based product comprises in the range of 0.1 to 100 ppm, such as 1-95 ppm, e.g. 10-90 ppm, such as 20-80 ppm, e.g. 30-70 ppm, such as 40-60 ppm, e.g. 35-55 ppm, such as 30-50 ppm, e.g. 25-45 ppm, preferably 20-40 ppm diacetyl.

An embodiment of the present invention relates to the product(s) as described herein, wherein the pH of the product(s) is in the range of 3.2 to 4.5, such as 3.3 to 4.4, e.g. 3.4 to 4.3, such as 3.5 to 4.2, e.g. 3.6 to 4.1, such as 3.7 to 4.0, e.g. 3.8 to 3.9 and preferably 3.2 to 3.7.

Another embodiments of the present invention relates to the product(s) as described herein, wherein the product(s) has a total sugar content in the range of 0.1 to 20 w/w%, such as 0.5 to 9.5 w/w%, e.g. 1 to 9 w/w%, such as 1.5 to 8.5 w/w%, e.g. 2 to 8 w/w%, such as 2.5 to 7.5 w/w%, e.g. 3 to 7 w/w%, such as 3.5 to 7.5 w/w%, e.g. 4 to 7 w/w%, such as 4.5 to 6.5 w/w%, e.g. 5 to 6 w/w%, such as 4.5 to 9.5 w/w%, and preferably the product has a total sugar content in the range of 1.5 %-3.0 w/w%, such as 1.7-2.5 w/w% total sugar.

Depending on the application of the products e.g. the fermented plant-based product a certain sweetness or lack of sweetness of the product(s) may be appreciated.

Yet, another embodiment of the present invention relates to the product(s) as described herein, wherein the plant base is selected from the group consisting of cereals, seeds, nuts, pulses, vegetables, stone fruits and combinations thereof. Another embodiment of the present invention relates to the product(s) as described herein, wherein the plant base is a cereal.

An further embodiment of the present invention relates to the product(s) as described herein, wherein the plant base is selected from the group consisting of oat, wheat, rye, barley, corn, rice, quinoa, chia, rapeseed, almond, coconut, carrot, pumpkin and combinations thereof. Yet, a further embodiment of the present invention relates to the product(s) as described herein, wherein the cereal is oat. If the fermented plant-based product is to be applied in a plant-based spreadable product oat as a plant base may be preferred.

Still, a further embodiment of the present invention relates to the product(s) as described herein, wherein the fermented plant-based product comprises at least one lactic bacterium. The benefits of having at least one lactic acid bacteria in the fermented plant-based product comprising diacetyl has been discussed above. In particular it may be the combination of lactic acid bacterial species that provides the unique flavour of the fermented plant-based product - diacetyl being one of the important flavouring components. In another embodiment one or more of the lactic bacteria produces diacetyl.

An embodiment of the present invention relates to the product(s) as described herein, wherein the lactic acid bacteria originate from one or more genera selected from the group consisting of *Lactobacillus, Lacticaseibacillus, Streptococcus, Bifidobacterium, Lactococcus, Leuconostoc* and combinations thereof.

Another embodiment of the present invention relates to the product(s) as described herein, wherein the lactic acid bacteria are selected from the group consisting of *Streptococcus thermophilus, Lactobacillus delbrueckii subsp. bulgaricus, Lacticaseibacillus paracasei, Lactobacillus acidophilus, Bifidobacterium lactis, Lactococcus lactis, Leuconostoc mesenteroides* and combinations thereof.

An embodiment of the present invention relates to the product(s) as described herein, wherein the lactic acid bacteria is *Streptococcus thermophilus.*

A further embodiment of the present invention relates to the product(s) as described herein, wherein the lactic acid bacteria is *Lactobacillus delbrueckii subsp. bulgaricus.*

Yet a further embodiment of the present invention relates to the product(s) as described herein, wherein the lactic acid bacteria *Lacticaseibacillus paracasei.*

A further embodiment of the present invention relates to the product(s) as described herein, wherein the lactic acid bacteria is *Lactobacillus acidophilus.*

Yet further embodiment of the present invention relates to the product(s) as described herein, wherein the lactic acid bacteria is *Bifidobacterium lactis.*

A further embodiment of the present invention relates to the product(s) as described herein, wherein the lactic acid bacteria is *Lactococcus lactis, Leuconostoc mesenteroides.*

Yet a further embodiment of the present invention relates to the product(s) as described herein, wherein the lactic acid bacteria is *Leuconostoc mesenteroides.*

Yet, another embodiment of the present invention relates to the product(s) as described herein, wherein the product(s) comprises *Streptococcus thermophilus* and *Lactobacillus delbrueckii subsp. bulgaricus.* Still, another embodiment of the present invention relates to the product(s) as described herein, wherein the product(s) comprises *Lacticaseibacillus paracasei, Lactobacillus acidophilus and Bifidobacterium lactis.*

A further embodiment of the present invention relates to the product(s) as described herein, wherein the product(s) comprises at least two lactic acid bacteria, preferably at least three lactic acid bacteria, more preferably at least four lactic acid bacteria, most preferably at least five lactic acid bacteria.

A further embodiment of the present invention relates to the product(s) as described herein, wherein the product(s) comprises more than five lactic acid bacteria.

In a preferred embodiment the product(s) comprises *Streptococcus thermophilus, Lactobacillus delbrueckii subsp. bulgaricus, Lacticaseibacillus paracasei, Lactobacillus acidophilus* and *Bifidobacterium lactis.*

It would be advantageous to apply the fermented plant-based product comprising diacetyl in a food product that can be used in vegan and vegetarian diets and be used as an ingredient in e.g. baking, cooking and confectionaries.

A further aspect of the present invention therefore relates to a food product comprising the fermented plant-based product as described herein.

Yet, one embodiment of the present invention relates to the food product as described herein, wherein the food product is be a dairy product, such as a cheese, a cream cheese, yogurts, drinkable products and a spreadable product. The food product may be an ingredient. Due to its butter-like flavour the fermented plant-based product comprising diacetyl may be used as an ingredient applied in bread and confectionary products.

Another embodiment of the present invention relates to the food product as described herein, wherein the food product is a dairy alternative product, such as cheese, cream cheese, yogurt, spreadable, drinkable dairy substitutes. In particular the dairy alternative product may be a product selected from the group consisting of plant-based butter-like product, plant-based spreadable product, a plant-based drinkable product and combinations thereof.

An embodiment of the present invention relates to a food product as described herein, wherein the product is a plant-based spreadable product. In the present context, the term "plant-based spreadable" is to be understood as a food product for human consumption comprising a firmness in the range of 200 to 600 load g at 5°C when measured by firmness analysis on a Texture Analyser (TA XT plus). In a specific embodiment the plant-based spreadable product comprises the fermented plant-based product comprising diacetyl according to the present invention. It may be preferred that the plant-based spreadable comprising the fermented plant-based product comprising diacetyl have a firmness in the range of 200 to 600 load g at 5°C when measured by firmness analysis.

An embodiment of the present invention relates to a food product as described herein, wherein the product is a plant-based butter-like product, e.g. a block product. In the present context, the term "butter-like product" is to be understood as a food product for human consumption comprising a firmness in the range of 500 to 1400 load g at 5°C when measured by firmness analysis on a Texture Analyser (TA XT plus). In a specific embodiment the plant-based butter-like product comprises the fermented plant-based product comprising diacetyl according to the present invention. It may be preferred that the plant-based butter-like product comprising the fermented plant-based product comprising diacetyl have a firmness in the range of 900 to 1200 load g at 5°C when measured by firmness analysis as described above.

In the present context, the terms "emulsion" is to be understood as a blend or suspension comprising a fat phase and a water phase. The emulsion may be an oil-in-water and/or a water-in-oil emulsion.

Another embodiment of the present invention relates to a food product as described herein. In a further embodiment the food product comprises a fat phase and a water phase in the form of an emulsion. The emulsion may be produced by emulsification of the fat phase and the water phase in any method available to the skilled person.

The food product of the present invention may comprise one or more ingredients selected from the group consisting of oils, fats, water, plant bases, salt, an acid, one or more colouring agents and any combination thereof.

Yet another aspect of the present invention relates to a process for producing a plant-based spreadable or butter-like product comprising diacetyl, wherein the process comprises the steps of:
a) providing an emulsion comprising:
   i. a fat phase,
   ii. a water phase comprising the fermented plant-based product as described herein,
b) heating the fat phase and water phase separately to at least 30°C, such as 40°C,
c) mixing the fat phase and water phase to form the emulsion,
d) heating the emulsion,
e) cooling the emulsion to at least 2 to 20°C, preferably 10°C to obtain the plant-based spreadable product comprising diacetyl,
f) obtaining a plant-based spreadable product.

A further embodiment of the present invention relates to the process as described herein, wherein the fat phase comprises at least one oil and/or at least one fat and at least one colouring agent.

Still, a further embodiment of the present invention relates to the process as described herein, wherein the water phase comprises water and at least one plant base and optionally at least one salt and further optionally at least one acid.

A further embodiment of the present invention relates to the process as described herein, wherein the emulsion is an oil-in-water emulsion and wherein the emulsion is an water-in-oil emulsion.

An embodiment of the present invention relates to the process as described herein, wherein the emulsion is heated to at least 70°C for at least 30 seconds under agitation and/or heated to at least 80°C for at least 15 seconds.

A specific embodiment of the present invention relates to a process for producing a plant-based spreadable or butter-like product comprising diacetyl, wherein the process comprises the steps of:
a) providing an emulsion comprising:
   i. a fat phase and at least one colouring agent,
   ii. a water phase comprising water, at least one plant-base, at least one acid the fermented plant-based product as described herein and optionally at least one salt,
b) heating the fat phase and water phase separately to at least 30°C , such as 40°C,
c) mixing the fat phase and water phase to form the emulsion,
d) heating the emulsion to at least 70°C for at least 30 seconds under agitation,
e) heating the emulsion to at least 80°C for at least 15 seconds,
f) cooling the emulsion to at least 2 to 20°C, preferably 10°C to obtain the plant-based spreadable product comprising diacetyl,
g) obtaining a plant-based spreadable product.

To obtain the desired firmness of the plant-based spreadable product it may be preferred that the at least one oil or fat is selected from the group consisting of almond-, avocado seed-, coconut-, corn-, cottonseed-, flax seed-, rapeseed-, hemp-, olive-, palm kernel-, peanut-, pumpkin seed-, rice bran-, safflower-, sesame seed-, sunflower seed-, soybean-, cashew-, hazelnut-, macadamia-, pecan-, pine nut-, pistachio-, walnut-, grapefruit seed- and shea oil and combinations thereof.

Thus, in a specific embodiment the at least one oil or fat is selected from rapeseed-, coconut-, corn-, sunflower seed-, palm kernel-, shea oil and combinations thereof.

Depending on the consumer and in particular depending on the geographical area where the plant-based spreadable is to be marketed various colouring agents may be applied. Thus, in at embodiment the at least one colouring agent is selected from the group consisting of approved colourants in the EU regulation 1333/2008, fungus produced pigments as natural β-carotene and pigments derived from plant sources such as pumpkin, carrot, beetroot, turmeric, safflower, Marigold, palm fruit, paprika, annatto, cochineal, sweet potato, berries, spirulina, green leaf plants and combinations hereof.

In a further embodiment the at least one colouring agent is selected from yellow to red colourants from the additives approved in the EU regulation 1333/2008, such as E160a carotenes, natural β-carotene, extracts of carrot, beetroot, sweet potato, annatto, pumpkin, palm fruit and combinations thereof.

In a further embodiment the plant-base may be selected from the group consisting of cereals, seeds, nuts, vegetables, stone fruits, pulses and combinations thereof. In a further embodiment the plant base is selected from the group consisting of oat, wheat, rye, barley, corn, rice, quinoa, chia, rapeseed, almond, coconut, carrot, pumpkin and combinations thereof. In a preferred embodiment the plant base is cereal. More specifically the cereal plant base may be selected from the group consisting of oat, wheat, rye, barley, corn, rice, quinoa and combinations thereof.

In a preferred embodiment the cereal is oat and this in an even more preferred embodiment the plant-baseis oat .

Again, depending on the consumer segment the choice of salt and in particular the amount of salt applied may differ. In a preferred embodiment the at least one salt is NaCl is preferable. It may be contemplated that the product(s) of the present invention comprises a salt content in the range of 0-10 w/w%, such as 0.5-9.5 w/w%, e.g. 1-9 w/w%, such as 1.5-8.5 w/w%, e.g. 2-8 w/w%, such as 2.5-7.5 w/w%, e.g. 3-7 w/w%, such as 3.5-6.5 w/w%, e.g. 4-6 w/w%, such as 4.5-5 w/w%.

In a further embodiment of the present invention the at least one acid is selected from the group consisting of fruit concentrates, juices, vinegar, and additives approved in the EU regulation 1333/2008 in the form of acidifiers in any form and combinations thereof.

In a further embodiment of the present invention the at least one acid is selected from the additives approved in the EU regulation 1333/2008, such as acetic acid, lactic acid, malic acid, fumaric acid, citric acid, phosphoric acid, metatartaric acid, sulphuric acid, gluconic acid, glutamic acid and/or fruit concentrates and juices, preferably citrus fruit and/or vinegars, preferably of neutral taste or with a taste where the products(s) would gain a benefit thereof.

An embodiment of the present invention relates to the process for producing a plant-based spreadable product comprising diacetyl as described herein, wherein the heating in step b) is performed for 1 second to 1, 2, 3, 4, 5 or 6 hours. It is however preferred that the heating in step b) is at least 1 minute, such as 2 minutes, such as 3 minutes, such as 4 minutes, such as 5 minutes, such as 10 minutes, such as 20 minutes, such as 30 minutes, such as 40 minutes, such as 50 minutes, such as 1 hour and preferably no more than 1 hour

It may be preferred that the heating step in step b) is performed at a temperature in the range of 30 to 70°C, such as 40 to 60, such as 42 to 58°C, e.g. 44 to 56°C, such as 46 to 54°C, e.g. 44 to 52°C, such as 46 to 50°C.

Due to the increasing demand from consumers for a plant-based spreadable product with a butter-like flavour similar to that of dairy products it would be advantageous to apply the fermented plant-based product comprising diacetyl as a flavour enhancing component in the plant-based spreadable product. Therefore, a further aspect of the present invention relates to a plant-based spreadable product comprising diacetyl obtainable by a process as described herein.

Still, a further aspect of the present invention relates to a plant-based spreadable product comprising diacetyl and/or more ingredients selected from the group consisting of oils, fats, water, a plant-base, salt, an acid, one or more colouring agents and any combination thereof.

An embodiment of the present invention relates to the plant-based spreadable product as described herein, wherein the diacetyl is provided from the fermented plant-based product according to process as described herein, and prepared according to the process as described herein.

Another embodiment of the present invention relates to the plant-based spreadable as described herein, wherein the plant-based spreadable product has a firmness in the range of 200 to 600 load g at 5°C when measured within 3 weeks after the initiation of the process for producing a plant-based spreadable product comprising diacetyl described herein. With respect to the present invention the product firmness was measured at 5 degrees C on a Texture Analyser (TA XT plus) one week after production. A cone (probe) having a 60 degree angle was pressed 8 mm into the undisturbed product. The trigger force was 5.0 g and the test speed was 49.8 mm per min. As the probe enters the sample, the force increases with the distance and the resistance to deformation by the applied stress is measured. The firmness (measured in gram) of the sample is the value of the maximum force applied to the sample over a specified distance (i.e. 8 mm).

Another aspect of the present invention relates to a use of the fermented plant-based product as described herein in the preparation of a food product as described herein, and wherein the fermented plant-based product as described herein is used as a flavour component in a food product.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "comprises", "having", "ranging", "range", "producing", "preparing", "prepared", "providing", "provided", "provide", "obtaining" and "obtained" are to be construed as openended terms (i.e., meaning "including, but not limited to,") unless otherwise noted. Recitation of ranges of values herein are merely intended to serve as a shorth and method for referring individually to each separate value falling within the range, unless otherwise indicated herein, and each separate value is incorporated into the specification as if it were individually recited herein. All processes and methods described herein can be performed in any suitable order unless otherwise indicated herein or otherwise clearly contradicted by context. The use of any and all examples, or exemplary language (e.g., "such as" and i.e.) provided herein, is intended merely to better illuminate the invention and does not pose a limitation on the scope of the invention unless otherwise claimed. No language in the specification should be construed as indicating any non-claimed element as essential to the practice of the invention.

The listing or discussion of an apparently prior published document in this specification should not necessarily be taken as an acknowledgement that the document is part of the state of the art or is common general knowledge.

Preferences, options and embodiments for a given aspect, feature or parameter of the invention should, unless the context indicates otherwise, be regarded as having been disclosed in combination with any and all preferences, options and embodiments for all other aspects, features and parameters of the invention. This is especially true for the description of the process for preparing the fermented plant-based product comprising diacetyl and all its features, which may readily be part of the fermented plant-based product comprising diacetyl obtained by the process and to the fermented plant-based product as such as described herein. Embodiments and features of the present invention are also outlined in the following items.

### Items

1. A process for preparing a fermented plant-based product comprising diacetyl, wherein the process comprises the steps of:
   a) provision of a mixture comprising:
      i. at least one plant base, and
      ii. at least one lactic acid bacterium,
   b) a first fermentation of the mixture until the pH of the mixture is in the range of 4.5 -6.5,
   c) addition of a substrate to the mixture of step b), and
   d) a second fermentation of the mixture of step c) to provide a fermented plant-based product comprising diacetyl.
2. The process according to item 1, wherein the substrate is pyruvate, pyruvate acid or salts thereof and/or citrate, citric acid or salts thereof.
3. The process according to items 1 or 2, wherein the substrate is citrate.
4. The process according to any one of the preceding items, wherein the substrate is pyruvate.
5. The process according to items 1 to 4, wherein the substrate is added in a concentration ranging from 0.01 to 0.5 g/L.
6. The process according to any one of the preceding items, wherein the mixture in step a) has a total sugar content in the range of 0.1 to 20 w/w%.
7. The process according to any one of the preceding items, wherein the plant base is selected from the group consisting of cereals, seeds, nuts, vegetables, stone fruits, pulses and combinations thereof.
8. The process according to any one of the preceding items, wherein the plant base is selected from the group consisting of oat, wheat, rye, barley, corn, rice, quinoa, chia, rapeseed, almond, coconut, carrot, pumpkin and combinations thereof.
9. The process according to any one of the preceding items, wherein the plant base is cereal.
10. The process according any one of the preceding items, wherein the cereal plant base is selected from the group consisting of oat, wheat, rye, barley, corn, rice, quinoa and combinations thereof.
11. The process according to any one of the preceding items, wherein the cereal is oat.
12. The process according to any one of the preceding items, wherein the form of the plant base is selected from the group consisting of a syrup, a paste, freeze dried, granular and combinations thereof.
13. The process according to any one of the preceding items, wherein the granular plant base is selected from a flour, a concentrate, an isolate and combinations thereof.
14. The process according to any one of the preceding items, wherein the mixture comprises a liquid medium.
15. The process according to any one of the preceding items, wherein the liquid medium is water.
16. The process according to any one of the preceding items, wherein the plant base is heated to at least 75 to 145°C prior to step a).
17. The process according to any one of the preceding items, wherein the plant base is heated for at least 0.3 seconds and for maximum 1 hour prior to step a).
18. The process according to any one of the preceding items, wherein one or more of the lactic bacteria produces diacetyl.
19. The process according to any one of the preceding items, wherein the lactic acid bacteria originate from one or more genera selected from the group consisting of *Lactobacillus, Lacticaseibacillus, Streptococcus, Bifidobacterium, Lactococcus, Leuconostoc* and combinations thereof.
20. The process according to any one of the preceding items, wherein the lactic acid bacteria are selected from the group consisting of *Streptococcus thermophilus, Lactobacillus delbrueckii subsp. bulgaricus, Lacticaseibacillus paracasei, Lactobacillus acidophilus, Bifidobacterium lactis, Lactococcus lactis, Leuconostoc mesenteroides* and combinations thereof.
21. The process according to any one of the preceding items, wherein the mixture comprises *Streptococcus thermophilus* and *Lactobacillus delbrueckii subsp. bulgaricus.*
22. The process according to any one of the preceding items, wherein the mixture comprises at least two lactic acid bacteria, preferably at least three lactic acid bacteria, more preferably at least four lactic acid bacteria, most preferably at least five lactic acid bacteria.
23. The process according to any one of the preceding items, wherein the mixture comprises *Streptococcus thermophilus, Lactobacillus delbrueckii subsp. bulgaricus, Lacticaseibacillus paracasei, Lactobacillus acidophilus, and Bifidobacterium lactis.*
24. The process according to any one of the preceding items, wherein the first and/or second fermentation is performed at a temperature in the range of 15 to 42°C.
25. The process according to any one of the preceding items, wherein the first fermentation is performed for 1 to 9 hours.
26. The process according to any one of the preceding items, wherein the second fermentation is performed for 2 to 16 hours.
27. The process according to any one of the preceding items, wherein the second fermentation is performed for no longer than 24 hours.
28. The process according to any one of the preceding items, wherein the second fermentation is terminated when the pH decreases to below 4.5.
29. The process according to any one of the preceding items, wherein said first fermentation is performed at least in part with agitation.
30. The process according to any one of the preceding items, wherein said fermented plant-based product comprises at least 1 ppm diacetyl.
31. The process according to item 31, wherein the fermented plant-based product comprises in the range of 1 to 100 ppm, such as 1-95 ppm, e.g. 10-90 ppm, such as 20-80 ppm, e.g. 30-70 ppm, such as 40-60 ppm, e.g. 35-55 ppm, such as 30-50 ppm, e.g. 25-45 ppm, preferably 20-40 ppm diacetyl.
32. A fermented plant-based product comprising diacetyl obtainable by a process according to any one of items 1 to 31.
33. A fermented plant based product comprising at least 1 ppm diacetyl.
34. The fermented plant-based product according to item 32-33, wherein the pH of the product is in the range of 3.2 to 4.5.
35. The fermented plant-based product according to any one of items 33 to 34, wherein the product has a total sugar content in the range of 0.01 to 20 w/w%.
36. The fermented plant-based product according to any one of the preceding items, wherein the plant base is selected from the group consisting of cereals, seeds, nuts, pulses, vegetables, stone fruits and combinations thereof.
37. The fermented plant-based product according to any one of the preceding items, wherein the plant base is a cereal.
38. The fermented plant-based product according to any one of the preceding items, wherein the plant base is selected from the group consisting of oat, wheat, rye, barley, corn, rice, quinoa, chia, rapeseed, almond, coconut, carrot, pumpkin and combinations thereof.
39. The fermented plant-based product according to any one of the preceding items, wherein the cereal is oat.
40. The fermented plant-based product according to any one of the preceding items, wherein said fermented plant-based product comprises at least one lactic bacterium.
41. The fermented plant-based product according to any one of the preceding items, wherein one or more lactic bacteria produces diacetyl.
42. The fermented plant-based product according to any one of the preceding items, wherein the lactic acid bacteria originate from one or more genera selected from the group consisting of *Lactobacillus, Lacticaseibacillus, Streptococcus, Bifidobacterium, Lactococcus, Leuconostoc* and combinations thereof.
43. The fermented plant-based product according to any one of the preceding items, wherein the lactic acid bacteria are selected from the group consisting of *Streptococcus thermophilus, Lactobacillus delbrueckii subsp. bulgaricus, Lacticaseibacillus paracasei, Lactobacillus acidophilus, Bifidobacterium lactis, Lactococcus lactis, Leuconostoc mesenteroides* and combinations thereof.
44. The fermented plant-based product according to any one of the preceding items, wherein said fermented plant-based product comprises *Streptococcus thermophilus* and *Lactobacillus delbrueckii subsp. bulgaricus.*
45. The fermented plant-based product according to any one of the preceding items, wherein said fermented plant-based product comprises at least three lactic acid bacteria, preferably at least four lactic acid bacteria, most preferably at least five lactic acid bacteria.
46. The fermented plant-based product according to any one of the preceding items, wherein said fermented plant-based product comprises *Streptococcus thermophilus, Lactobacillus delbrueckii subsp. bulgaricus, Lacticaseibacillus paracasei, Lactobacillus acidophilus, and Bifidobacterium lactis.*
47. A food product comprising the fermented plant-based product according to items 32 and/or 46.
48. The food product according to item 47, wherein the food product is a dairy alternative product.
49. The food product according to item 48, wherein the dairy alternative product is a product selected from the group consisting of plant-based butter-like product, plant-based spreadable product and a plant-based drinkable product.
50. The food product according to any one of items 47 to 49, wherein the food product is a dairy product, such as a cheese, a cream cheese, yogurts, drinkable products and a spreadable product.
51. The food product according to any one of the preceding items, wherein the food product is an ingredient.
52. The food product according to any one of the preceding items, wherein the product is a plant-based spreadable product.
53. The food product according to any one of the preceding items, wherein said food product comprises a fat phase and a water phase in the form of an emulsion.
54. The food product according to any one of the preceding items, wherein the food product comprises one or more ingredients selected from the group consisting of oils, fats, water, a plant-base, salt, an acid, one or more colouring agents and any combination thereof.
55. A process for producing a plant-based spreadable product comprising diacetyl, wherein the process comprises the steps of:
   a) providing an emulsion comprising:
      i. a fat phase,
      ii. a water phase comprising the fermented plant-based product as described herein,
   b) heating the fat phase and water phase separately to at least 40°C,
   c) mixing the fat phase and water phase to form the emulsion,
   d) heating the emulsion,
   e) cooling the emulsion to at least 2 to 20°C, preferably 10°C to obtain the plant-based spreadable product comprising diacetyl,
   f) obtaining a plant-based spreadable product.
56. The process according to item 55, wherein the fat phase comprises at least one oil and/or at least one fat.
57. The process according to item 55, wherein the fat phase comprises at least one colouring agent.
58. The process according to item 55, wherein the water phase comprises water.
59. The process according to item 55, wherein the water phase comprises at least one plant base.
60. The process according to item 55, wherein the water phase comprises at least one salt.
61. The process according to item 55, wherein the water phase comprises at least one acid.
62. The process according to item 55, wherein the heating in step b) is performed for at least 0 minutes, such as 1 minute, such as 2 minutes, such as 3 minutes, such as 4 minutes, and such as 5 minutes.
63. The process according to item 55, wherein the emulsion is an oil-in-water emulsion.
64. The process according to item 55, wherein the emulsion is an water-in-oil emulsion.
65. The process according to item 55, wherein the emulsion is heated to at least 70°C for at least 30 seconds under agitation.
66. The process according to item 55, wherein the emulsion is heated to at least 80°C for at least 15 seconds.
67. A plant-based spreadable product obtainable by a process according to any one of items 55 to 64.
68. A plant-based spreadable product comprising diacetyl and or more ingredients selected from the group consisting of oils, fats, water, a plant-base, salt, an acid, one or more colouring agents and any combination thereof.
69. A plant-based spreadable product comprising diacetyl according to item 60, wherein the diacetyl is provided from the fermented plant-based product according to items 32 to 46, and prepared according to items 1 to 31.
70. A plant-based spreadable product according to any one of the preceding items, wherein the plant-based spreadable product has a firmness in the range of 200 to 600 load g at 5°C.
71. A plant-based spreadable product according to any one of the preceding items, wherein the plant-based spreadable product is obtained after 3 weeks.
72. Use of the fermented plant-based product according to any one of items 32 to 46 and/or 47 to 54 in the preparation of a food product.
73. Use of the fermented plant-based product according to items 32 to 46 as a flavour enhancing component in a food product.

### Examples

### Example 1: Process for preparing a fermented plant-based product comprising diacetyl

70-200 ml oat plant-base is diluted in deionized water. The total volume of oat plant-base and deionized water is 1L. The oat medium is heated at 75-95°C for 1-5 min and then cooled down on ice. Once the oat medium reaches a temperature below 30°C a lactic acid bacterial culture is inoculated into the medium. A total of 1-150 mg of lactic acid bacterial culture is added to obtain 1L of oat medium. The culture is incubated in a water bath at 25-30°C. After 2-5 hours at 25-30°C 0.1-1.5g substrate (0.05 % citrate or pyruvate) is added. After at least 15-24 hours a fermented plant-based product comprising diacetyl is obtained. The diacetyl content is measured by head space gas chromatography-mass spectrometry (GC-MS)

### Example 2: Process for preparing a plant based spreadable product

Providing the fermented plant-based product comprising diacetyl of Example 1.

Preparing a fat phase by providing an oil liquid at ambient temperatures and heating it. Mixing the heated liquid oil with an oil or fat being solid at room temperatures that has been pre-heated. Adding colourant if oil soluble to the mixture and mixing. Obtaining a fat phase.

Preparing a water phase by providing pre-heated water. Adding any plant base for flavouring to the heated water and mixing. Adding the fermented plant-based product comprising diacetyl and mixing. Adding colourant if water soluble. Adding salt if a product containing salt is desired and acid if a more acidic product is desired and mixing. Obtaining water phase.

Heating the fat phase and the water phase separately to a temperature where all fat is melted.

Mixing the heated fat phase and heated water phase under intensive agitation at and creating an emulsion. Heating the emulsion to >50 °C under intensive mixing.

Cooling the mixture from >50 °C to >5 °C and obtaining a plant based spreadable product.

## Claims

1. A process for preparing a fermented plant-based product comprising diacetyl, wherein the process comprises the steps of:
a) provision of a mixture comprising:
i. at least one plant base, and
ii. at least one lactic acid bacterium,
b) a first fermentation of the mixture until the pH of the mixture is in the range of 4.5 - 6.5,
c) addition of a substrate to the mixture, and
d) a second fermentation of the mixture to provide a fermented plant-based product comprising diacetyl.

2. The process according to claim 1, wherein the substrate is pyruvate, pyruvate acid or salts thereof and/or citrate, citric acid or salts thereof.

3. The process according to any one of the preceding claims, wherein the mixture in step a) has a total sugar content in the range of 0.1 to 20 w/w%.

4. The process according to any one of the preceding claims, wherein the plant base is oat.

5. The process according to any one of the preceding claims, wherein the lactic acid bacteria are selected from the group consisting of *Streptococcus thermophilus, Lactobacillus delbrueckii subsp. bulgaricus, Lacticaseibacillus paracasei, Lactobacillus acidophilus, Bifidobacterium lactis, Lactococcus lactis, Leuconostoc mesenteroides, Leuconostoc pseudomesenteroides* and combinations thereof.

6. The process according to any one of the preceding claims, wherein the mixture comprises *Streptococcus thermophilus, Lactobacillus delbrueckii subsp. bulgaricus, Lacticaseibacillus paracasei, Lactobacillus acidophilus* and *Bifidobacterium lactis.*

7. A fermented plant-based product comprising diacetyl obtainable by a process according to any one of claims 1-6.

8. A fermented plant based product, said product comprising at least 1 ppm diacetyl.

9. The fermented plant-based product according to any one of claims 7-8, wherein said fermented plant-based product comprises *Streptococcus thermophilus, Lactobacillus delbrueckii subsp. bulgaricus, Lacticaseibacillus paracasei, Lactobacillus acidophilus* and *Bifidobacterium lactis.*

10. A food product comprising the fermented plant-based product according to any one of claims 7-9.

11. A process for producing a plant-based spreadable product comprising diacetyl, wherein the process comprises the steps of:
a) providing an emulsion comprising:
i. a fat phase,
ii. a water phase comprising the fermented plant-based product according to claims 7-9,
b) heating the fat phase and water phase separately to at least 40°C,
c) mixing the fat phase and water phase to form the emulsion,
d) heating the emulsion,,
e) cooling the emulsion to at least 2 to 20°C, to obtain the plant-based spreadable product comprising diacetyl,
f) obtaining a plant-based spreadable product.

12. A plant-based spreadable product obtainable by a process according to claim 11.

13. A plant-based spreadable product comprising diacetyl and or more ingredients selected from the group consisting of oils, fats, water, a plant-base, salt, an acid, one or more colouring agents and any combination thereof.

14. Use of the fermented plant-based product according to any one of claims 7-9 in the preparation of a food product.

15. Use of the fermented plant-based product according to any one of claims 7-9 as a flavour enhancing component in a food product.
